# EUROPÄISCHE PATENTSCHRIFT

(11) **EP 2 210 572 B1**
(45) Veröffentlichungstag und Bekanntmachung des Hinweises auf die Patenterteilung: **10.02.2016**
(21) Anmeldenummer: 09015272.9
(22) Anmeldetag: 10.12.2009
(51) Int. Cl.: A61F 2/18

(54) **Passive Gehörknöchelchenprothese mit Applikator**
Passive ossicular prosthetic with applicator
Prothèse de l'osselet de l'oreille passive avec applicateur

(30) Priorität: 24.01.2009 DE 102009006047
(43) Veröffentlichungstag der Anmeldung: 28.07.2010
(73) Patentinhaber: Heinz Kurz GmbH Medizintechnik, 72144 Dusslingen (DE)
(72) Erfinder: Steinhardt, Uwe, 72145 Hirrlingen (DE); Kurz, Heinz, 72144 Dusslingen (DE); àWengen, Daniel, Felix, Dr., 4102 Binningen (CH)
(74) Vertreter: Kohler Schmid Möbus Patentanwälte

(56) Entgegenhaltungen:
- US-A- 5 171 240

## Beschreibung

Die Erfindung betrifft eine Gehörknöchelchenprothese, die mindestens ein Glied der menschlichen Gehörknöchelchenkette ersetzt oder überbrückt, wobei die Gehörknöchelchenprothese an einem Ende ein erstes Befestigungselement zur mechanischen Verbindung mit dem Trommelfell oder einem Glied der Gehörknöchelchenkette, insbesondere mit dem Ambossfortsatz oder mit dem Hammergriff, und an ihrem anderen Ende ein zweites Befestigungselement zur mechanischen Verbindung mit einem weiteren Glied oder Teilen eines Gliedes der Gehörknöchelchenkette oder direkt mit dem Innenohr sowie ein die beiden Befestigungselemente Schall leitend miteinander verbindendes, längliches Verbindungselement umfasst.

Derartige Gehörknöchelchenprothesen dienen zur Verbesserung der Schallübertragung bei unterschiedlichen pathologischen Befunden. Sie werden verwendet, um bei ganz oder teilweise fehlenden oder geschädigten Gehörknöchelchen des menschlichen Mittelohrs den Schall vom Trommelfell zum Innenohr zu übertragen. Die Gehörknöchelchenprothese weist dabei zwei Enden auf, wobei je nach den konkreten Gegebenheiten das eine Ende der Gehörknöchelchenprothese zum Beispiel mittels einer Kopfplatte am Trommelfell befestigt und das andere Ende der Gehörknöchelchenprothese beispielsweise am Steigbügel der menschlichen Gehörknöchelchenkette befestigt oder direkt ins Innenohr getaucht wird.

Drei besonders häufig verwendete Arten von Gehörknöchelchenprothesen sind die Steigbügel-Prothesen, die Partial-Prothesen und die Total-Prothesen. Steigbügel-Prothesen (=Stapes-Prothesen) werden am Amboss oder am Hammergriff fixiert und ragen über einen Stempel (=Piston) ins Innenohr. Partial-Prothesen liegen meist mit einer Kopfplatte am Trommelfell an und stellen eine Verbindung zum Steigbügelköpfchen her. Total-Prothesen verbinden das Trommelfell mit der Steigbügel-Fußplatte.

Die maximale Länge solcher implantierbaren passiven Gehörknöchelchenprothesen liegt in der Größenordnung von nur wenigen Millimetern, was die praktische Handhabung der Prothesen schwierig gestaltet. Zum operativen Einsetzen in das menschliche Mittelohr benutzen Chirurgen in der Regel neben dem Mikroskop geeignete Spezialinstrumente wie Zängchen, Pinzetten, feine Nadeln, Mikrosauger und dergleichen.

In der US 6,892,466 B2 wird ein spezieller Applikator gezeigt, der zum Einsetzen von Prothesenattrappen ins Mittelohr für die Ermittlung der aktuellen Größe einer für den jeweiligen Patienten individuell benötigten Gehörknöchelchenprothese dienen soll. Da die dort beschriebenen Prothesenattrappen in Form und Größe mit den einzusetzenden Gehörknöchelchenprothesen möglichst gut übereinstimmen sollen, wäre ein solcher Applikator natürlich prinzipiell auch zum Einsetzen der eigentlichen Prothesen geeignet.

Die US 5,171,240 beschreibt eine Vorrichtung mit der Merkmalen der Oberbegriffs des Anspruchs 1.

Nachteilig bei Verwendung dieser bekannten Applikatoren ist jedoch, dass sie vor der Operation - wie auch jedes andere chirurgische Instrument - aufwändig desinfiziert und gründlich sterilisiert werden müssen. Des Weiteren sind diese Applikatoren in der Regel nicht in Form und Größe speziell auf die jeweils gerade verwendete Gehörknöchelchenprothese abgestimmt, so dass von vornherein mehrere unterschiedliche Applikatoren bereit gestellt werden müssen, wenn die Möglichkeit zur individuellen Auswahl einer bestimmten Prothese aus dem recht großen vorhandenen Sortiment offengehalten werden soll. Andererseits muss sich dann aber der Chirurg während der Operation schnell entscheiden, welchen der bereitgestellten Applikatoren er für das Handling der zum Einsetzen ins Mittelohr des Patienten ad hoc bestimmten Prothese benutzen will, was oftmals zu einer nicht optimalen Auswahl des Werkzeugs und damit zu einer weiteren Komplizierung der ohnehin nicht trivialen Implantationsoperation führt.

Daraus kann sich ein weiteres, sogar noch gravierenderes Problem ergeben: Aufgrund der kleinen Abmessungen muss eine Operation im Mittelohr zwangsläufig unter dem Mikroskop erfolgen. Dieses wird vom Operateur während der Eröffnungsphase auf das Operationsfeld, also den Gehörgang bzw. das Mittelohr gerichtet. Wenn dann irgendwann im Verlauf der Operation die Gehörknöchelchenprothese eingesetzt werden soll, bleibt natürlich das Mikroskop zumeist weiterhin auf den zuletzt betrachteten Bereich des Mittelohrs scharf gestellt, während die Prothese aus ihrer sterilen Lager- und Transport-Verpackung herausgeholt wird. Für diesen Zweck bedienen sich die meisten Operateure eines in der Regel nicht dafür optimierten Instruments und auch nicht des - auf das Operationsfeld eingestellten - Mikroskops, sondern führen insbesondere den Vorgang des Greifens der Prothese ohne Vergrößerung "nach Gefühl und mit bloßem Auge" durch. Dabei wird die Gehörknöchelchenprothese oftmals beschädigt oder gar zerstört, so dass ein weiteres Implantat in den Bereich des Gehörgangs bzw. des Mittelohrs gebracht werden muss.

Das Greifen und die Handhabung einer so winzigen Vorrichtung wie einer Gehörknöchelchenprothese und insbesondere deren punktgenaue Platzierung und Ausrichtung am Zielort im Mittelohr, sollte aber mit höchster feinmechanischer Präzision und vor allem routinemäßig und standardisiert durchgeführt werden.

Aufgabe der vorliegenden Erfindung ist es demgegenüber, eine gattungsgemäße passive Gehörknöchelchenprothese der eingangs beschriebenen Art mit möglichst einfachen technischen Mitteln dahin gehend zu verbessern, dass sie vom Chirurgen in standardisierter Weise aus der üblichen sterilen Verpackung entnommen und sofort - ohne aufwändige Vorbehandlung und anschließende Bereitstellung weiterer Hilfsmittel - unmittelbar in den Gehörgang des Patienten verbracht und ins Mittelohr implantiert werden kann, wobei eine möglichst optimale, individuell auf die Geometrie der jeweils ausgewählten Prothese abgestimmte Handhabung während der Implantationsoperation gewährleistet und insbesondere eine Beschädigung allein aufgrund des Greifens und des Transports der Prothese nahezu ausgeschlossen sein soll.

Erfindungsgemäß wird diese Aufgabe auf ebenso überraschend einfache wie wirkungsvolle Art und Weise dadurch gelöst, dass ein länglicher Applikator zum Transport der Gehörknöchelchenprothese von einer Sterilverpackung zum Operationsfeld und Einbringen ins Mittelohr oder den Gehörgang vorgesehen ist, welcher einerseits ein von der Gehörknöchelchenprothese weg ragendes freies Ende zur Handhabung sowie andererseits einen Eingriffsteil aufweist, der zunächst kraftschlüssig, formschlüssig oder über eine abbrechbare oder abscherbare Materialbrücke an der Gehörknöchelchenprothese befestigt ist, und nach dem Einsetzen der Gehörknöchelchenprothese ins Mittelohr oder den Gehörgang von der Gehörknöchelchenprothese abgetrennt und mitsamt dem Applikator aus dem Mittelohr oder dem Gehörgang entfernt werden kann.

Dies ermöglicht unaufwändig und kostengünstig die Bereitstellung einer aus der sterilen Verpackung bereits operationsfertig entnehmbaren Einheit aus einer Gehörknöchelchenprothese und einem optimal auf deren Geometrie angepassten, individuellen Transportmittel. Der Applikator kann nach der Implantation der Prothese problemlos aus dem Mittelohr entfernt und entsorgt werden, was die praktische Handhabung der erfindungsgemäßen Gehörknöchelchenprothese für den Chirurgen enorm erleichtert. Der gesamte Vorgang ist standardisiert und außer dem Operationsmikroskop sind keine weiteren Hilfsmittel erforderlich, insbesondere keine sterile Vorbehandlung derselben.

Besonders bevorzugt sind Ausführungsformen der Erfindung, bei denen der Applikator am ersten Befestigungselement der Gehörknöchelchenprothese befestigt ist. Diese Anordnung ist schon ergonomischen Gründen besonders günstig, weil die exakte Platzierung gerade des ersten Befestigungselements im Mittelohr für die endgültige Lage der Prothese im implantierten Zustand entscheidend ist. Außerdem wird das erste Befestigungselement in der Regel der dem Außenohr zunächst gelegene Teil der Prothese sein, weshalb sich von hier aus der Applikator nach Abschluss der Implantation am leichtesten aus dem Mittelohr entfernen lässt.

Bei einer ersten Klasse von Ausführungsformen der Erfindung ist der Applikator mit seinem Eingriffsteil an der Gehörknöchelchenprothese über eine abbrechbare oder abscherbare Materialbrücke befestigt, die eine Sollbruchstelle aufweist, an welcher das Material des Applikators im Querschnitt verjüngt ist. Diese Geometrie lässt sich fertigungstechnisch relativ einfach und preiswert herstellen. Allerdings wird nach dem Abbrechen oder Abscheren des Eingriffsteils oftmals ein spitzer Rest der Materialbrücke an der Gehörknöchelchenprothese stehen bleiben. Diese kleine Spitze muss von vornherein an einer unkritischen Stelle der Gehörknöchelchenprothese angeordnet sein, wo hinterher keine Gefahr einer Verletzung oder Beschädigung der Umgebung durch den Brückenrest besteht.

Eine alternative Klasse von Ausführungsformen, bei der dieses Problem prinzipiell nicht auftritt, zeichnet sich dadurch aus, dass der Eingriffsteil des Applikators einen fest mit der Gehörknöchelchenprothese verbundenen Andockteil kraftschlüssig und/oder formschlüssig umgreift. Freilich ist hier der Fertigungsaufwand etwas höher als bei der Herstellung einer simplen Materialbrücke. Dafür eröffnen sich hierdurch aber sehr viel mehr Möglichkeiten zur individuellen Platzierung und Gestaltung des Andockbereiches.

Besonders einfach in der Fertigung und in der Handhabung sind Weiterbildungen dieser Klasse von Ausführungsformen, bei denen der Andockteil in dem Bereich, in welchem er vom Eingriffsteil des Applikators umgriffen wird, eine Verdickung aufweist, die insbesondere kugelig geformt ist.

Vorzugsweise weist der Eingriffsteil die Form einer Klammer oder eines Clips auf, um einen reversiblen Kraftschluss zwischen dem Eingriffsteil und dem Andockteil herzustellen.

Ganz besonders bevorzugt sind Varianten der Erfindung, bei denen der Applikator zumindest im Bereich des Eingriffsteils aus einem Material mit Formgedächtnis (=memory effect), insbesondere aus Nitinol hergestellt ist. Die Verwendung derartiger Materialien ist auf dem Gebiet der Gehörknöchelchenprothesen an sich bekannt, erweist sich aber gerade im Zusammenhang mit der vorliegenden Erfindung als besonders wirkungsvoll. Auf diese Weise kann nach dem endgültigen Platzieren der Gehörknöchelchenprothese im Mittelohr durch einfache Zufuhr von Wärme eine thermische Aktivierung des Eingriffsteils erfolgen und damit ohne mechanischen Eingriff seine Form gezielt so verändert werden, dass der Kraftschluss zwischen dem Eingriffsteil und dem Andockteil gelöst wird. Der Applikator kann dann problemlos aus dem Mittelohr entfernt werden.

Bei einer weiteren Klasse von Ausführungsformen der erfindungsgemäßen Gehörknöchelchenprothese, die ebenfalls das Problem von möglicherweise spitzen oder scharfkantigen Brückenresten vermeiden, wird der Eingriffsteil des Applikators von einem fest mit der Gehörknöchelchenprothese verbundenen Aufnahmeteil kraftschlüssig und/oder formschlüssig umgriffen.

Fertigungstechnisch besonders einfach und preisgünstig herstellbare Weiterbildungen zeichnen sich dadurch aus, dass der Aufnahmeteil als eine in Richtung auf den Eingriffsteil des Applikators hin offene Hülse ausgebildet ist.

Besonders bevorzugt sind Varianten dieser Weiterbildungen, bei denen der Aufnahmeteil eine zylindrische oder konische Bohrung aufweist, in welche ein entsprechend geformter konischer Endabschnitt des Eingriffsteils eingreifen kann.

Andere Varianten weisen einen in Richtung auf den Eingriffsteil des Applikators hin offenen V-förmigen Spalt als Aufnahmeteil auf, in welchen ein keilförmiger Endabschnitt des Eingriffsteils eingreifen kann.

Bei einer anderen Gruppe von Weiterbildungen der oben beschriebenen Ausführungsform weist der Eingriffsteil eine Verdickung auf, die vom Aufnahmeteil klammerartig umgriffen wird. Der Aufnahmeteil ist zumindest im Bereich des Eingriffsteils aus einem Material mit Formgedächtnis, insbesondere aus Nitinol hergestellt. Auch bei dieser Variante der Erfindung kann die bereits oben im Zusammenhang mit anderen Ausführungsformen beschriebene thermische Aktivierung erfolgen, indem mittels Wärmezufuhr zum Aufnahmeteil eine vorbestimmte Formänderung hervorgerufen und dadurch der Kraft- oder Formschluss zwischen dem Eingriffsteil und dem Andockteil aufgehoben wird.

Alternativ kann bei weiteren einfachen und geometrisch besonders kompakten Varianten das erste Befestigungselement als Klammer oder Clip geformt sein und auf seiner Innenseite einen als Aufnahmeteil zur Aufnahme des Eingriffsteils ausgebildeten Bereich aufweisen.

Je nach gewünschtem Anwendungsgebiet und dem individuellen Defekt, der bei einem Patienten durch den Einsatz der erfindungsgemäßen Gehörknöchelchenprothese behoben oder zumindest in seinen Auswirkungen gelindert werden soll, kann die erfindungsgemäße Gehörknöchelchenprothese mechanisch sehr unterschiedliche Ausprägungen aufweisen: So kann etwa ein als Klemmteil wirkendes Befestigungselement Klammerartig, Manschettenartig, Hülsenartig, Clipartig, Spangenartig, Klemmringartig oder Gürtelartig aufgebaut sein. Der Grundgedanke der vorliegenden Erfindung ist damit im Bereich der Mittelohr-Prothetik ziemlich universell einsetzbar.

Bei vielen Ausführungsformen kann beispielsweise die Prothese einerseits am Ambossfortsatz oder am Steigbügel befestigt sein oder direkt ins Innenohr getaucht werden. Vorteilhaft ist in diesem Zusammenhang eine Ausgestaltung, bei der die Gehörknöchelchenprothese am Endpunkt des Hammers (=Umbo) oder direkt daneben angeordnet ist, wodurch die größte Hebelwirkung für die mechanische Übertragung des Schalls durch Bewegungen in der künstlichen oder natürlichen Gehörknöchelchenkette erzielt wird.

Eine Klasse von Ausführungsformen der erfindungsgemäßen Gehörknöchelchenprothese zeichnet sich dadurch aus, dass das zweite Befestigungselement als Platte, als Hülse, als Schlinge, als geschlossene Glocke, als einfach oder mehrfach geschlitzte Glocke oder als Clip zur mechanischen Verbindung mit einem weiteren Glied der Gehörknöchelchenkette ausgebildet ist.

Bei Weiterbildungen dieser Ausführungsformen ist die Prothese über ein als Kopfplatte ausgebildetes erstes Befestigungselement einerseits am Trommelfell und über das zweite Befestigungselement andererseits am Amboss oder am Steigbügel befestigt.

Alternative Ausgestaltungen können vorsehen, dass die Gehörknöchelchenprothese an ihrem das zweite Befestigungselement tragenden Ende mittels Perforation der Steigbügelfußplatte (=Stapedektomie bzw. Stapedotomie) und/oder mittels Eröffnung der menschlichen Hörschnecke (=Cochleotomie) direkt an das Innenohr angekoppelt ist, insbesondere über einen Kolben.

Nachdem die Prothese operativ im Mittelohr platziert wurde und das Trommelfell wieder verschlossen ist, beginnt die so genannte Einheilphase. In dieser Zeit bilden sich Narben und diese verursachen unvorhersehbar Kräfte, welche dazu führen können, die Prothese aus ihrer lokalen Position zu verschieben. Aus diesem Grund ist es sehr hilfreich, wenn die Prothese eine gewisse post-operative Mobilität aufweist, so dass sich die Kopfplatte postoperativ selbstständig der Position des Trommelfells angleichen kann. Da zudem die anatomischen Gegebenheiten des Ohrs, wie beispielsweise die Lage, die Form und die Größe des Steigbügels, des Ambosses, des Hammers und des Trommelfells variieren, ist es sehr vorteilhaft, wenn Gehörknöchelchenprothesen nicht völlig starr ausgebildet sind, sondern eine gewisse Flexibilität oder Variabilität aufweisen.

In der Regel wird bei der erfindungsgemäßen GehörknöchelchenProthese das Verbindungselement zwischen den beiden Befestigungselementen als länglicher Schaft gestaltet sein, wie dies an sich aus dem Stand der Technik wohlbekannt ist. Um die oben erörterte erhöhte Flexibilität bzw. Variabilität der Prothese zu erreichen - wie beispielsweise ausführlich in der EP 1 181 907 B1 beschrieben - kann bei einer besonders bevorzugten Ausführungsform der Erfindung am oder im länglichen Schaft mindestens ein Kugelgelenk vorgesehen sein. Vorteilhaft im Hinblick auf eine besonders hohe postoperative Beweglichkeit der Prothese sind Weiterbildungen, bei denen der längliche Schaft eine Vielzahl von aneinander angrenzenden weiteren Drehelementen, vorzugsweise eine Kugelgelenkkette, umfasst.

Neben der postoperativen Positionsverschiebung ergibt sich nach der Implantation von Gehörknöchelchenprothesen auch noch ein weiteres Problem: Das Mittelohr des menschlichen Körpers stellt nämlich ein "halb offenes Lager" dar. Jedes Implantationsmaterial, welches im Rahmen einer Rekonstruktion des Mittelohres und seiner Strukturen in den Körper eingebracht wird, erfährt dadurch eine besondere Beanspruchung, dass eine kontaminierte und infizierte Umgebung vorherrscht, die in der Regel das Material angreift. Da das Ziel der Implantation einer Gehörknöchelchenprothese immer auch eine möglichst lange, komplikationsfreie Verweildauer des Implantats im Mittelohr des Patienten sein muss, kann ein lange andauernder Materialangriff zu Beschädigungen der Prothese und/oder zu einer lokalen Infektion führen. Beide Folgen sind nicht tolerabel. Um eine Schädigung sowohl des Implantationsmaterials als auch des umgebenden Gewebes dauerhaft zu verhindern, ist bei einer weiteren besonders bevorzugten Ausführungsform der Erfindung die Oberfläche der Gehörknöchelchenprothese ganz oder zumindest abschnittsweise mit einer biologisch aktiven Beschichtung, insbesondere einer wachstumshemmenden und/oder einer wachstumsfördernden und/oder einer antibakteriell wirkenden Beschichtung überzogen.

Ein als Trommelfell-Kopfplatte ausgebildetes erstes Befestigungselement sollte grundsätzlich eine wachstumsfördernde Beschichtung, ein direkt ins Innenohr führendes, etwa in Form eines Kolbens ausgebildetes zweites Befestigungselement hingegen immer eine wachstumshemmende Beschichtung aufweisen.

Die erfindungsgemäße Gehörknöchelchenprothese selbst oder Teile davon können aus Titan und/oder aus Gold und/oder aus Tantal und/oder aus Stahl und/oder aus einer Legierung der genannten Metalle hergestellt sein. Insbesondere das Material Titan weist neben seiner Festigkeit und ausgezeichneten Schallleitungseigenschaften bekanntermaßen auch eine hervorragende Biokompatibilität am menschlichen Mittelohr auf.

Vorteilhaft im Hinblick auf die oben erwähnte postoperative Lageanpassung sind Ausführungsformen der Erfindung, bei denen die Gehörknöchelchenprothese selbst oder Teile davon, insbesondere eines der Befestigungselemente, aus einem Material mit dem - oben diskutierten - Formgedächtnis (=memory effect) oder superelastischen Eigenschaften, vorzugsweise aus Nitinol hergestellt sind, was per se etwa aus der WO 02/069850 A1 oder der US 6,554,861 B2 bekannt ist.

Alternativ oder ergänzend können bei weiteren Ausführungsformen Teile der erfindungsgemäßen Gehörknöchelchenprothese aus einem Keramikmaterial hergestellt sein.

Möglich sind aber auch Ausführungsformen der Erfindung, bei denen die gesamte Prothese oder Teile davon aus biokompatiblen Kunststoffen, insbesondere Silikon, Polytetrafluorethylen (PTFE) oder Faserverbundwerkstoffen hergestellt sind. Mit diesen Materialien können post-operative Abstoßungsreaktionen in den meisten Fällen ebenfalls verhindert werden.

Besonders bevorzugt ist eine Ausführungsform der erfindungsgemäßen Gehörknöchelchenprothese, bei der die Massenverteilung der einzelnen Teile der Prothese in Abhängigkeit von einem gewünschten, vorgebbaren Frequenzgang der Schallleitung im Mittelohr berechnet ist. Damit lässt sich ohne großen zusätzlichen technischen Aufwand gewissermaßen ein Tuning der Schallfortpflanzungseigenschaften mittels einer individuellen ausgestalteten Gehörknöchelchenprothese erreichen.

Ein solcher Tuning Effekt kann bei speziellen Ausführungsformen beispielsweise dadurch erzielt werden, dass mindestens eine zusätzliche Masse in Abhängigkeit von einem gewünschten, vorgebbaren Frequenzgang der Schallleitung im Mittelohr an einem Teil der Gehörknöchelchenkette bzw. der Prothese befestigt ist. Bei vorteilhaften Weiterbildungen dieser Ausführungsformen ist die zusätzliche Masse mittels eines Clips an einem Teil der Gehörknöchelchenkette oder der Prothese befestigt. Außerdem können die zusätzliche Masse und/oder der Clip ebenfalls mit einer biologisch aktiven Beschichtung überzogen sein.

Eine weitere Ausführungsform der Erfindung zeichnet sich schließlich dadurch aus, dass die Prothese mit einem aktiven Vibrationsteil eines aktiven, insbesondere implantierbaren Hörgeräts verbunden ist. Damit lassen sich auch weitergehende Gehörschäden durch Einsatz moderner Elektronik in weiten Bereichen beheben oder zumindest in ihren Auswirkungen wesentlich lindern, wobei eine körperliche Verbindung der Prothese mit der Außenwelt aufgrund der oben beschriebenen Beschichtung wiederum keine Probleme durch einen erhöhten Bakterieneintrag in den Bereich des Mittelohres verursacht, wenn die Beschichtung entsprechend antibakteriell ausgestaltet ist.

Weitere Merkmale und Vorteile der Erfindung ergeben sich aus der nachfolgenden detaillierten Beschreibung von Ausführungsbeispielen der Erfindung anhand der Figuren der Zeichnung, die erfindungswesentliche Einzelheiten zeigt, sowie aus den Ansprüchen. Die einzelnen Merkmale können je einzeln für sich oder zu mehreren in beliebigen Kombinationen bei Varianten der Erfindung verwirklicht sein.

In der schematischen Zeichnung sind Ausführungsbeispiele der Erfindung dargestellt, welche in der nachfolgenden Beschreibung näher erläutert werden.

Es zeigen:
- Fig. 1a: eine schematische räumliche Darstellung einer ersten Ausführungsform der erfindungsgemäßen Gehörknöchelchenprothese mit einer Trommelfell-Kopfplatte als erstem Befestigungselement, an welchem über eine Materialbrücke der Applikator anhängt;
- Fig. 1b: eine Ausführungsform mit einem Clip als erstem Befestigungselement, an welchem über eine abscherbare Materialbrücke der Applikator anhängt;
- Fig. 2a: eine Ausführungsform mit einer Klammer als erstem Befestigungselement, welche einen Andockteil mit Verdickung aufweist, der von einem zunächst aufgespreizten clipförmigen Eingriffsteil aus Material mit Formgedächtnis umgriffen wird, wobei nach Anwendung einer thermischen Behandlung das clipförmige Eingriffsteil um das Andockteil geschlossen wurde;
- Fig. 2b: eine Ausführungsform mit einer Klammer als erstem Befestigungselement, welche einen Andockteil mit Verdickung aufweist, der von einem klammerförmigen Eingriffsteil umgriffen wird;
- Fig. 3: eine Ausführungsform mit einer Klammer als erstem Befestigungselement, das einen V-förmigen Spalt als Aufnahmeteil aufweist, in welchen ein keilförmiger Endabschnitt des Eingriffsteils des Applikators eingreift
- Fig. 4a: eine Ausführungsform mit einer Trommelfell-Kopfplatte als erstem Befestigungselement, die einen hülsenförmigen Aufnahmeteil mit Bohrung aufweist, in welchen ein konischer Endabschnitt des Eingriffsteils des Applikators eingreift;
- Fig. 4b: die Ausführungsform nach Fig. 4a mit aus dem Aufnahmeteil des ersten Befestigungselements herausgezogenem Applikator;
- Fig. 4c: den Applikator der Figuren 4a und 4b in größerem Detail; und
- Fig. 5: eine Ausführungsform mit einer Klammer als erstem Befestigungselement, die auf ihrer Innenseite einen als Aufnahmeteil zur formschlüssigen Aufnahme des Eingriffsteils des Applikators ausgebildeten Bereich aufweist.

Die in den Figuren der Zeichnung schematisch dargestellten - im Detail unterschiedlich gestalteten - Ausführungsformen der erfindungsgemäßen **Gehörknöchelchenprothese 10; 10'; 20; 20'; 30; 40; 50** weisen jeweils am einen Ende jeweils ein **erstes Befestigungselement 11; 11'; 21; 21'; 31; 41; 51** auf, welches der mechanischen Verbindung der Prothese mit dem Trommelfell oder einem Glied der Gehörknöchelchenkette, insbesondere mit dem Ambossfortsatz oder mit dem Hammergriff, dient. Am anderen Ende der Gehörknöchelchenprothese 10; 10'; 20; 20'; 30; 40; 50 sitzt jeweils ein **zweites Befestigungselement 12; 12'** zur mechanischen Verbindung der Prothese mit einem weiteren Glied oder Teilen eines Gliedes der Gehörknöchelchenkette, beispielsweise über eine **Glocke 12** mit der Steigbügelfußplatte, oder zum direkten Eintauchen über einen **Kolben 12'** ins Innenohr. Dazwischen ist ein die beiden Befestigungselemente 11; 11'; 21; 21'; 31; 41; 51 bzw. 12; 12' Schall leitend miteinander verbindendes **Verbindungselement 13; 13'; 13"** angeordnet, welches bei allen in der Zeichnung gezeigten Ausführungsformen in Form eines länglichen Schaftes ausgeführt ist.

Erfindungsgemäß ist jeweils **ein länglicher Applikator 14; 14'; 24; 24'; 34; 44; 54** zum Transport der Gehörknöchelchenprothese 10; 10'; 20; 20'; 30; 40; 50 von einer Sterilverpackung zum Operationsfeld und Einbringen ins Mittelohr oder den Gehörgang vorgesehen, welcher einerseits ein von der Gehörknöchelchenprothese weg ragendes **freies Ende 15; 15'; 25; 25'; 35; 45; 55** zur Handhabung sowie andererseits einen **Eingriffsteil 16; 16'; 26; 26'; 36; 46; 56** aufweist, der zunächst kraftschlüssig, formschlüssig oder über eine abbrechbare oder abscherbare Materialbrücke an der Gehörknöchelchenprothese befestigt ist, und nach dem Einsetzen der Gehörknöchelchenprothese ins Mittelohr oder den Gehörgang von der Gehörknöchelchenprothese abgetrennt und mitsamt dem Applikator aus dem Mittelohr oder dem Gehörgang entfernt werden kann.

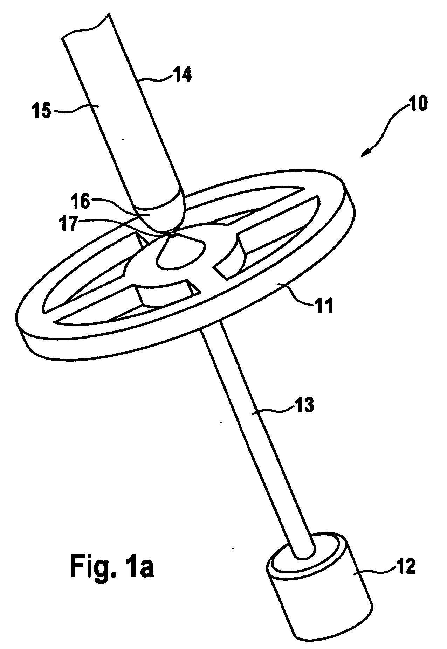

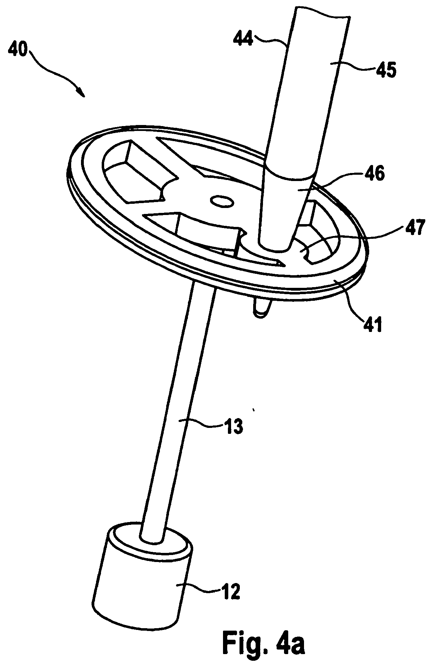

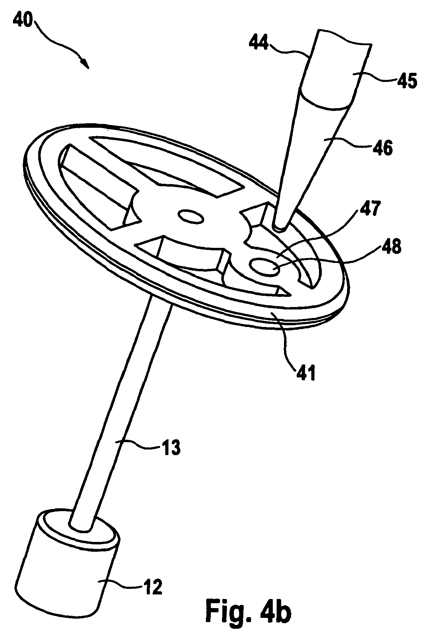

Bei den in den Figuren 1a, 4a und 4b gezeigten Ausführungsformen ist das erste Befestigungselement 11; 41 jeweils in Form einer Kopfplatte zur Anlage am Trommelfell ausgebildet. Das zweite Befestigungselement 12 ist hier glockenartig gestaltet und dient vorzugsweise zur Befestigung der Gehörknöchelchenprothese 10 am Steigbügel.

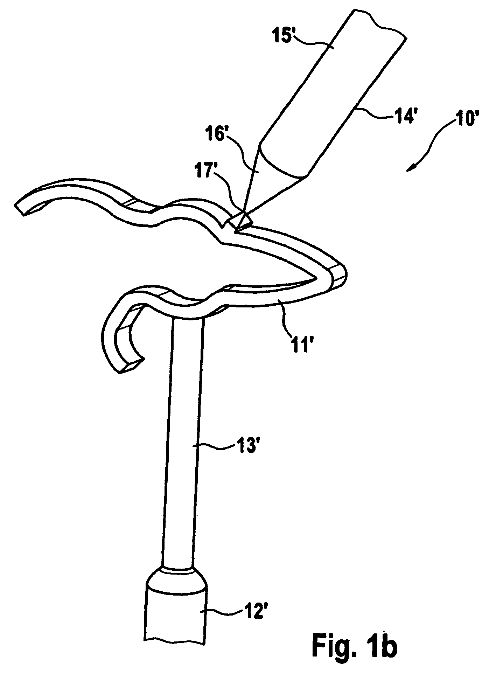

Bei den Ausführungsformen nach den Figuren 1b, 2a, 2b, 3 und 5 hingegen ist in das erste Befestigungselement 11'; 21; 21'; 31; 51 in Form einer Klammer ausgebildet, die beispielsweise auf den AmbossFortsatz oder auch auf ein anderes Glied der Gehörknöchelchenkette aufgeclipst werden kann. Das zweite Befestigungselement 12' an dem der Kopfplatte entgegen gesetzten Ende ist in diesen Ausführungsbeispielen jeweils als Kolben zur direkten Ankopplung der Gehörknöchelchenprothese 10'; 20; 20'; 30; 50 an das Innenohr gestaltet.

Stattdessen könnte das zweite Befestigungselement bei in der Zeichnung nicht dargestellten Ausführungsformen der erfindungsgemäßen Gehörknöchelchenprothese auch anders gestaltet sein, etwa als Platte, als Hülse, als Schlinge, als vorzugsweise rund gebogener, teilweise offener Haken, als geschlossene Glocke, als einfach oder mehrfach geschlitzte Glocke, als Klammer oder als Clip.

Die in den Figuren 1a und 1b gezeigten Ausführungsformen weisen jeweils einen Applikator 14; 14' auf, der mit seinem Eingriffsteil 16; 16' an der Gehörknöchelchenprothese 10; 10' über eine abbrechbare oder abscherbare Materialbrücke befestigt ist, die eine **Sollbruchstelle 17; 17'** besitzt, an welcher das Material des Applikators 14; 14' im Querschnitt verjüngt ist.

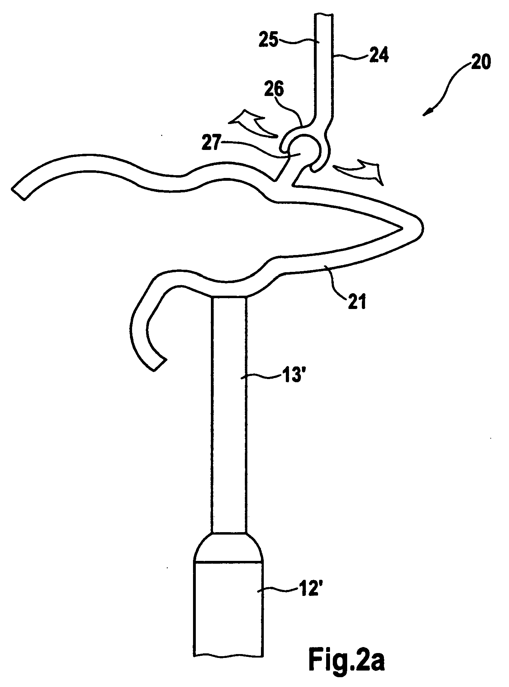

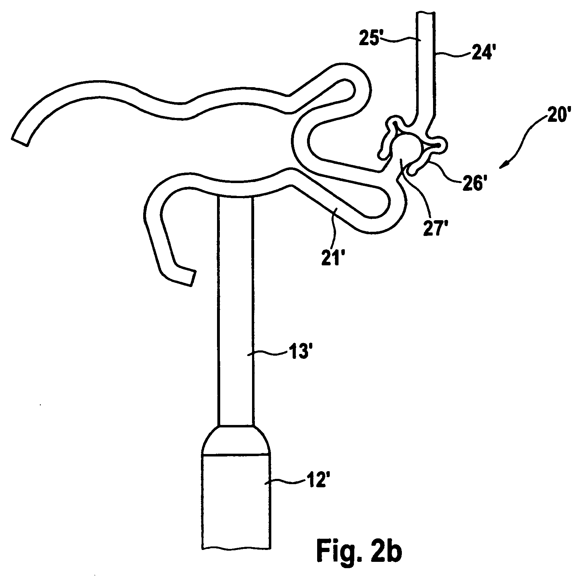

Die in den Figuren 2a und 2b gezeigten Ausführungsformen zeichnen sich dadurch aus, dass der Eingriffsteil 26; 26' des Applikators 24; 24' einen fest mit der Gehörknöchelchenprothese 20; 20' verbundenen **Andockteil 27; 27'** aufweist, der bei diesen Ausführungsbeispielen jeweils eine kugelige Verdickung in dem Bereich, in welchem er vom Eingriffsteil 26; 26' des Applikators 24; 24' umgriffen wird besitzt, und der kraftschlüssig und/oder formschlüssig vom Eingriffsteil 26; 26' umgriffen wird.

Der Applikator 24 kann zumindest im Bereich des Eingriffsteils 26 aus einem Material mit Formgedächtnis (=memory effect), insbesondere aus Nitinol hergestellt sein. Die Pfeile in Figur 2a deuten an, dass der clipförmige Eingriffsteils 26 nach dem Einsetzen der Gehörknöchelchenprothese 20 an ihre gewünschte Position im Ohr dann durch simplen Wärmeeintrag aufgespreizt werden kann, um den Applikator 24 an seinem freien Ende 25 aus dem Ohr herauszuziehen.

Der Eingriffsteil 26' des Applikators 24' in Figur 2b ist in Form einer Klammer ausgebildet, die auf den kugeligen Andockteil 27' mit geringer Anpresskraft aufgeclipst ist und die nach dem Einbringen der Gehörknöchelchenprothese 20' in den Gehörgang des Patienten oder nach ihrer endgültigen Positionierung im Mittelohr leicht vom ersten Befestigungselement 21' abgezogen werden kann, um dann den Applikator 24' zu entfernen.

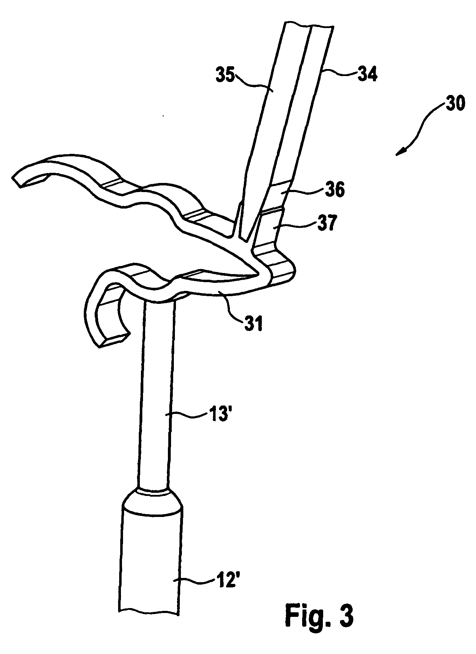

Bei den in den Figuren 3 bis 5 dargestellten Ausführungsformen wird der Eingriffsteil 36; 46; 56 des Applikators 34; 44; 54 bis zur endgültigen Positionierung Gehörknöchelchenprothese 30; 40; 50 im Mittelohr oder bis zum Ablegen derselben im Gehörgang von einem fest mit der Prothese verbundenen **Aufnahmeteil 37; 47; 57** kraftschlüssig und/oder formschlüssig umgriffen.

Die in Figur 3 gezeigte Gehörknöchelchenprothese 30 weist an ihrem ersten Befestigungselement 31 einen Aufnahmeteil 37 auf, der als ein in Richtung auf den Eingriffsteil 36 des Applikators 34 hin offener V-förmiger Spalt ausgebildet ist, in welchen bis zur Ablage oder Positionierung der Prothese im Ohr ein keilförmiger Endabschnitt des Eingriffsteils 36 leicht klemmend eingreift. Dieser kann danach problemlos mit dem freien Ende 35 des Applikators 34 abgezogen werden.

Bei der in den Figuren 4a bis 4c dargestellten Ausführungsform ist der Aufnahmeteil 47 als eine in Richtung auf den Eingriffsteil 46 des Applikators 44 hin offene Hülse ausgebildet, die eine zylindrische oder konische **Bohrung 48** besitzt, in welche zunächst ein konischer Endabschnitt des Eingriffsteils 46 leicht klemmend eingreift (Figur 4a), der später zur Entfernung des Applikators 44 leicht herausgezogen werden kann (Figur 4b).

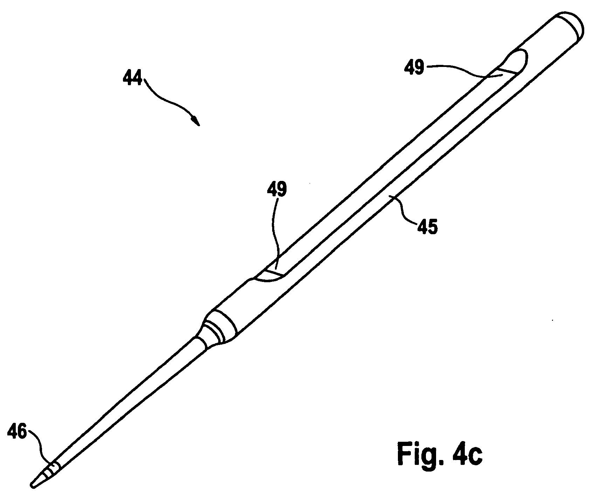

Figur 4c zeigt den stiftförmigen Applikator 44 in größerem Detail mit seinem spitz zulaufenden Eingriffsteils 46 und einem freien Ende 45, das eine ergonomisch günstige einseitige **Ausnehmung 49** zur Verbesserung der Handhabung des Applikators 44 aufweist.

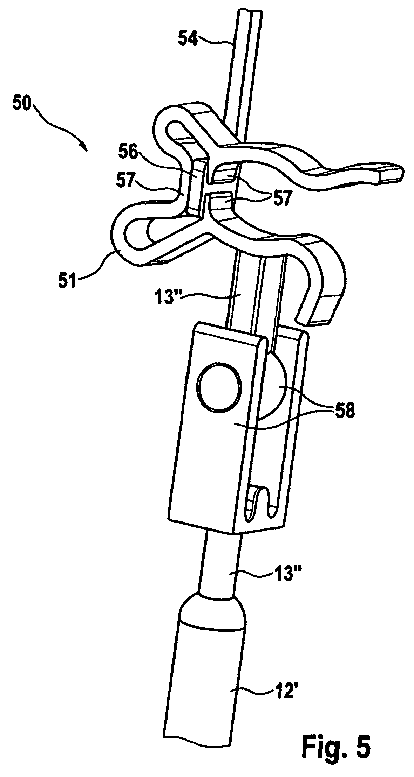

In der Ausführungsform nach den Figur 5 schließlich ist in das Verbindungselement 13" ein **Kugelgelenk 58** integriert, um eine gewisse postoperative Flexibilität der Gehörknöchelchenprothese 50 zwischen ihren Verbindungsstellen zu erreichen. Das erste Befestigungselement 51 ist hier als Klammer geformt. Es weist auf seiner Innenseite einen als Aufnahmeteil 57 zur Aufnahme des Eingriffsteils 56 ausgebildeten Bereich auf, aus dem der Eingriffsteil 56 nach Positionierung der Gehörknöchelchenprothese 50 im Ohr problemlos gelöst werden kann, um den Applikator 54 zu entfernen.

Bei in der Zeichnung nicht eigens dargestellten Ausführungsformen der erfindungsgemäßen Gehörknöchelchenprothese kann der Eingriffsteil des Applikators eine Verdickung aufweisen, die vom Aufnahmeteil des ersten Befestigungselements klammerartig umgriffen wird, wobei der Aufnahmeteil zumindest im Bereich des Eingriffsteils aus einem Material mit Formgedächtnis (=memory effect), insbesondere aus Nitinol hergestellt ist. Nach der Platzierung der Gehörknöchelchenprothese im Ohr kann dann der Eingriffsteil durch Wärmeeintrag aus dem Aufnahmeteil gelöst und der Applikator entfernt werden.

Die Massenverteilung der einzelnen Teile der erfindungsgemäßen Gehörknöchelchenprothese 10; 10'; 20; 20'; 30; 40; 50 kann in Abhängigkeit von einem gewünschten, vorgebbaren Frequenzgang der Schallleitung im Mittelohr derart berechnet sein, dass ein individuelles Tuning der Schallleitungs-Eigenschaften ermöglicht wird. Bei in der Zeichnung nicht dargestellten Ausführungsformen kann mindestens eine zusätzliche Masse in Abhängigkeit von einem gewünschten, vorgebbaren Frequenzgang der Schallleitung im Mittelohr an der Gehörknöchelchenprothese oder an einem Teil der Gehörknöchelchenkette befestigt sein, insbesondere mittels eines Clips.

## Patentansprüche

1. Vorrichtung umfassend eine Gehörknöchelchenprothese (40) und einen länglichen Applikator (44), wobei die mindestens ein Glied der menschlichen Gehörknöchelchenkette ersetzende oder überbrückende, Prothese an einem Ende ein erstes Befestigungselement (41) zur mechanischen Verbindung mit dem Trommelfell oder einem Glied der Gehörknöchelchenkette, und an ihrem anderen Ende ein zweites Befestigungselement (12) zur mechanischen Verbindung mit einem weiteren Glied oder Teilen eines Gliedes der Gehörknöchelchenkette oder direkt mit dem Innenohr sowie ein die beiden Befestigungselemente (41 bzw. 12) Schall leitend miteinander verbindendes, längliches Verbindungselement (13) umfasst, wobei der längliche Applikator (44) zum Transport der Gehörknöchelchenprothese (40) von einer Sterilverpackung zum Operationsfeld und Einbringen ins Mittelohr oder den Gehörgang vorgesehen ist, und einerseits ein von der Gehörknöchelchenprothese (40) weg ragendes freies Ende (45) zur Handhabung sowie andererseits einen Eingriffsteil (46) aufweist, und wobei der Eingriffsteil (46) des Applikators (44) von einem fest mit der Gehörknöchelchenprothese (40) verbundenen Aufnahmeteil (47) kraftschlüssig und/oder formschlüssig umgriffen ist, **dadurch gekennzeichnet, dass** der Aufnahmeteil (47) als eine in Richtung auf den Eingriffsteil (46) des Applikators (44) hin offene Hülse ausgebildet ist, die eine zylindrische oder konische Bohrung (48) aufweist, in welche ein konischer Endabschnitt des Eingriffsteils (46) eingreifen kann.

2. Vorrichtung nach Anspruch 1, **dadurch gekennzeichnet, dass** der Applikator (44) am ersten Befestigungselement (41) der Gehörknöchelchenprothese (40) befestigt ist.

3. Vorrichtung nach Anspruch 1 oder 2, **dadurch gekennzeichnet, dass** der Eingriffsteil eine Verdickung aufweist, die vom Aufnahmeteil klammerartig umgriffen wird, und dass der Aufnahmeteil zumindest im Bereich des Eingriffsteils aus einem Material mit Formgedächtnis (=memory effect), insbesondere aus Nitinol hergestellt ist.

4. Vorrichtung nach einem der Ansprüche 1 bis 3, **dadurch gekennzeichnet, dass** das erste Befestigungselement (41) in Form einer Trommelfell-Kopfplatte zur Anlage gegen das Trommelfell ausgebildet ist, und dass der Applikator (44) an der im implantierten Zustand der Gehörknöchelchenprothese (40) dem Trommelfell zugewandten Seite der Trommelfell-Kopfplatte befestigt ist, insbesondere am Zentrum der Trommelfell-Kopfplatte auf der dem Angriffspunkt des länglichen Verbindungselements (13) gegenüber liegenden Seite.

## Claims

1. A device comprising an ossicular prosthesis (40) and an elongate applicator (44), in which case the prosthesis, designed to replace or bridge at least one element of the human ossicular chain, comprises a first fastening element (41) at one end for mechanical connection to the tympanic membrane or an element of the ossicular chain, a second fastening element (12) at its other end for mechanical connection to another element or parts of an element of the ossicular chain or for direct connection to the inner ear, and an elongate connecting element (13) which joins together the two fastening elements (41 and 12) in a sound-conducting manner, and in which case the elongate applicator (44), provided for transferring the ossicular prosthesis (40) from a sterile package to the operation site and inserting it into the middle ear or auditory canal, has a first free end (45) extending away from the ossicular prosthesis (40) for handling purposes, and at its other end an engagement portion (46), said engagement portion (46) of the applicator (44) being gripped in a force- and/or form-locking manner by a receiving portion (47) connected to the ossicular prosthesis (40), **characterized in that** the receiving portion (47) takes the form of a sleeve which is open in direction of the engagement portion (46) of the applicator (44) and which has a cylindrical or conical bore (48) in which a conical end section of the engagement portion (46) can engage.

2. A device according to Claim 1, **characterized in that** the applicator (44) is attached to the first fastening element (41) of the ossicular prosthesis (40).

3. A device according to Claim 1 or 2, **characterized in that** the engagement portion has a thickened area which is enclosed in a clamp-like manner by the receiving portion, and **in that**, at least in the region of the engagement portion, the receiving portion is made of a material with shape memory (memory effect), in particular nitinol.

4. A device according to one of Claims 1 to 3, **characterized in that** the first fastening element (41) takes the form of a headplate for placement against the tympanic membrane, and **in that** the applicator (44) is attached to the side of the headplate that faces the tympanic membrane in the implanted state of the ossicular prosthesis (40) and in particular to the centre of the tympanic membrane headplate on the side opposite the point of attachment of the elongate connecting element (13).

## Revendications

1. Dispositif, comprenant une prothèse pour osselets de l'oreille oblong (40) et un applicateur (44), dans lequel la prothèse remplaçant ou contournant au moins un organe de la chaîne des osselets de l'oreille humaine comprend, à une extrémité, un premier élément de fixation (41) pour le raccordement mécanique au tympan ou à un organe de la chaîne des osselets de l'oreille et, à son autre extrémité, un deuxième élément de fixation (12) pour le raccordement mécanique à un autre organe ou à des parties d'un organe de la chaîne des osselets de l'oreille ou directement à l'oreille interne, ainsi qu'un élément de raccordement oblong (13) raccordant l'un à l'autre, avec conduction du son, les deux éléments de fixation (41 ou respectivement 12), dans lequel l'applicateur oblong (44) est prévu pour le transport de la prothèse pour osselets de l'oreille (40) à partir d'un emballage stérile vers le champ opératoire et pour sa mise en place dans l'oreille moyenne ou dans le conduit auditif, et présente d'une part une extrémité libre (45) dépassant de la prothèse pour osselets de l'oreille (40) pour la manipulation ainsi que d'autre part une partie de prise (46), et dans lequel la partie de prise (46) de l'applicateur (44) est entourée, par complémentarité de forces et/ou par complémentarité de formes, par une partie de réception (47) raccordée fixement à la prothèse pour osselets de l'oreille (40), **caractérisé en ce que** la partie de réception (47) est constituée en tant que douille, ouverte en direction de la partie de prise (46) de l'applicateur (44), laquelle douille présente un alésage cylindrique ou conique (48) dans lequel un tronçon d'extrémité conique de la partie de prise (46) peut s'engrener.

2. Dispositif selon la revendication 1, **caractérisé en ce que** l'applicateur (44) est fixé sur le premier élément de fixation (41) de la prothèse pour osselets de l'oreille (40).

3. Dispositif selon la revendication 1 ou 2, **caractérisé en ce que** la partie de prise présente un épaississement qui est entouré à la façon d'une agrafe par la partie de réception, et **en ce que** la partie de réception est, au moins dans la zone de la partie de prise, réalisée dans un matériau à mémoire de forme (= memory effect), en particulier en nitinol.

4. Dispositif selon l'une des revendications 1 à 3, **caractérisé en ce que** le premier élément de fixation (41) est constitué sous la forme d'une plaque de tête de tympan destinée à venir s'appuyer contre le tympan, et **en ce que** l'applicateur (44) est fixé sur le côté de la plaque de tête de tympan qui, dans l'état implanté de la prothèse pour osselets de l'oreille (40), est tourné vers le tympan, en particulier au centre de la plaque de tête de tympan sur le côté situé en face du point d'attaque de l'élément de raccordement oblong (13).
